# FASCICULE DE BREVET EUROPEEN

(11) **EP 3 194 132 B1**
(45) Date de publication et mention de la délivrance du brevet: **06.11.2019**
(21) Numéro de dépôt: 15763235.7
(22) Date de dépôt: 28.08.2015
(51) Int. Cl.: D06M 13/342, D06M 13/35, D06M 13/422, B27K 3/15, C08L 97/02

(54) **MATERIAU ISOLANT THERMIQUE A BASE D'AEROGEL**
WÄRMEDÄMMSTOFF AUF DER BASIS VON AEROGEL
HEAT INSULATION MATERIAL BASED ON AEROGEL

(30) Priorité: 28.08.2014 FR 1458092
(43) Date de publication de la demande: 26.07.2017
(73) Titulaire: Université de Lorraine, 54052 Nancy Cedex (FR); Centre National de la Recherche Scientifique (C.N.R.S.), 75016 Paris (FR)
(72) Inventeur: JAMART, Brigitte, F-54500 Vandoeuvre (FR); DEGIOVANNI, Alain, F-54000 Nancy (FR); SON, Sébastien, F-54820 Marbache (FR); JANNOT, Yves, F-55190 Troussey (FR); PICKAERT, Guillaume, F-54220 Malzéville (FR)
(74) Mandataire: IPAZ
(86) Numéro de dépôt international: PCT/EP2015/069762
(87) Numéro de publication internationale: WO 2016/030513

(56) Documents cités:
- EP-A1- 2 281 961
- WO-A2-2010/133798
- WO-A2-2010/143902
- US-A1- 2002 094 426
- BROSSE N ET AL: "A family of strong low-molecular-weight organogelators based on aminoacid derivatives", TETRAHEDRON LETTERS, PERGAMON, GB, vol. 45, no. 52, 20 décembre 2004 (2004-12-20), pages 9521-9524, XP004727391, ISSN: 0040-4039, DOI: 10.1016/J.TETLET.2004.10.139

## Description

La présente invention concerne un matériau isolant thermique à base d'aérogel.

Les aérogels sont définis comme des gels secs possédant généralement des pores de volume nanométrique. Ce genre de matériaux est obtenu par séchage supercritique d'organogels ce qui permet d'éliminer le solvant tout en conservant la texture poreuse du gel humide.

Les Inventeurs ont décrit précédemment dans la demande WO2010/133798 une série d'organogélateurs dérivés d'acides aminés naturels, permettant de former des gels physiques organiques de faible masse moléculaire. Les aérogels obtenus à partir de ces organogélateurs par extraction du solvant sont des matériaux nanostructurés et mésoporeux qui présentent des propriétés remarquables en termes notamment de surface spécifique, de très faible contribution du solide et de stabilité thermique dans le temps en raison de sa très grande hydrophobicité.

Cependant, les aérogels décrits dans la demande WO2010/133798 n'ont pas la résistance mécanique ni la densité requises pour être utilisés seuls pour certaines applications, notamment comme matériau isolant dans le bâtiment.

Il existe par conséquent le besoin de développer des matériaux isolants thermiques ayant à la fois les propriétés des aérogels décrits dans WO2010/133798, notamment l'hydrophobicité, et une meilleure rigidité et résistance physique.

La demande US2002/0094426 décrit un composite comprenant un aérogel et une matrice de support, par exemple une ouate, obtenu par la mise en œuvre de l'extraction au CO₂ supercritique sur ladite matrice de support imprégnée d'une solution d'un matériau capable de former un gel inorganique, tel qu'un gel de silice, ou un gel de polymère.

Etant donné que ces gels sont des gels chimiques maintenus par liaisons covalentes et irréversibles, leur utilisation pour imprégner une matrice présente certains inconvénients. En effet, une fois que la gélification est faite, il n'est plus possible de corriger le défaut survenu lors de l'imprégnation de la matrice de support. Par ailleurs, les composites décrits dans cette demande ont besoin d'un traitement chimique supplémentaire pour rendre les produits finaux hydrophobes. De plus, la polymérisation des gels de polymère nécessite également l'ajout de compléments chimiques.

Il y a donc un intérêt industriel à proposer des matériaux isolants thermiques qui puissent être fabriqués plus facilement.

L'invention a pour objectif de pallier ces manques techniques et de proposer un tel matériau isolant thermique.

En menant des recherches sur les aérogels décrits dans WO2010/133798, les Inventeurs ont constaté que, contre toute attente, un matériau isolant thermique satisfaisant à ces exigences peut être obtenu par séchage d'une matrice fibreuse imprégnée par les organogélateurs décrits dans cette demande.

Le matériau de l'invention obtenu après le séchage est très hydrophobe et exempté des traitements supplémentaires qui sont nécessaires pour le composite décrit dans US2002/0094426. C'est d'autant plus surprenant que le matériau de l'invention présente une meilleure résistance physique et une plus grande densité par rapport à la susdite matrice fibreuse non imprégnée.

L'invention concerne un matériau isolant thermique obtenu par séchage d'une matrice fibreuse imprégnée par une solution de pseudopeptides de formule (I), 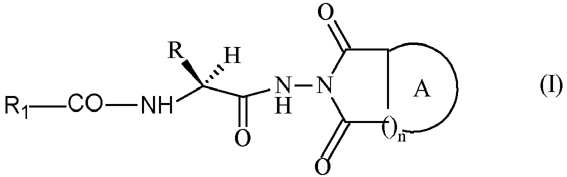 dans laquelle :
- R représente une chaîne latérale d'un acide aminé naturel ou synthétique,
- R₁ représente soit un groupe (C₁-C₈)alkyle droit ou ramifié, soit un groupe (C₁-C₈)alcoxy droit ou ramifié, soit un groupe aryle, soit un groupe aryle(C₁-C₄)alkyle, soit un groupe aryloxy, soit un hétérocycle saturé ou insaturé, n = 1 ou 2 et
- A représente un groupe aromatique ou hétéroaromatique à un ou plusieurs cycles,
ladite matrice fibreuse ayant une faible conductivité thermique inférieure à 0,05 W/m/K et une densité inférieure à 50kg/m³.

La conductivité thermique du produit final est mesurée par des méthodes bien connues de l'homme du métier, en utilisant par exemple, la méthode du plan chaud centré (Y. Jannot, V. Felix, A. Degiovanni, Measurement Science and Technology 2010, 21, n° 035106) ou la méthode du tricouche (Y. Jannot, G. Payet, A. Degiovanni, IJHMT 2009, 52, 1105-1111), complétée par une mesure au plan chaud centré (Y. Jannot, V. Felix, A. Degiovanni, Measurement Science and Technology 2010, 21, n° 035106).

La préparation du matériau de l'invention repose sur la pénétration totale de la solution de pseudopeptides de formule (I) dans la matrice fibreuse et la formation préalable d'un produit intermédiaire « organogel/matrice », dans lequel toutes les cavités de la matrice fibreuse sont comblées par l'organogel formé par les pseudopeptides de formule (I).

Etant donné que ces organogels sont des gels physiques et thermoréversibles, une fois que la matrice fibreuse est imprégnée de la solution de pseudopeptides de formule (I), le produit intermédiaire « organogel/matrice » peut être obtenu simplement par refroidissement. Par ailleurs, le défaut de l'imprégnation apparu sur le produit intermédiaire peut être corrigé facilement par réchauffement.

Au sens de la présente invention, on entend par (C₁-C₈)alkyle droit ou ramifié une chaîne hydrocarbonée de 1 à 8 atomes de carbone, notamment de 1 à 6 atomes de carbone. Comme par exemple les groupes : méthyle, éthyle, propyle, isopropyle, butyle, isobutyle, sec-butyle, tert-butyle, pentyle, isopentyle, néopentyle, tert-pentyle, hexyle, isohexyle, 1,1-diméthylbutyle, 2,2-diméthylbutyle, 3,3- diméthylbutyle and 2-éthylbutyle.

Au sens de la présente invention, on entend par acide aminé naturel ou synthétique, notamment les acides aminés suivants : l'acide aspartique (Asp ou D), l'asparagine (Asn ou N), la thréonine (Thr ou T), la sérine (Ser ou S), l'acide glutamique (Glu ou E), la glutamine (Gln ou Q), la glycine (Gly ou G), l'alanine (Ala ou A), la cystéine (Cys ou C), la valine (Val ou V), la méthionine (Met ou M), l'isoleucine (Ile ou I), la leucine (Leu ou L), la tyrosine (Tyr ou Y), la phénylalanine (Phe ou F), l'histidine (His ou H), la lysine (Lys ou K), le tryptophane (Trp ou W), la proline (Pro ou P) et l'arginine (Arg ou R).

Au sens de la présente invention, on entend par aryle, un groupe choisi dans le groupe comprenant phényle, benzyle, tolyle, xylyle et naphtyle.

Au sens de la présente invention, on entend par hétérocycle saturé ou insaturé ou par hétéroromatique un groupe choisi dans le groupe comprenant oxiranyle, azétidinyle, oxétanyle, thiétanyle, pyrrolidinyle, tétrahydrofuryle, thiolanyle, pipéridyle, tétrahydropyranyle, morpholinyle, thiomorpholinyle et pipérazinyle, pyridyle, pyrimidinyle, pyridazinyle, pyrrolyle, furanyle, thiophényle, imidazolyle, pyrazolyle, isoxazolyle, isothiazolyle, thiazolyle, oxazolyle, 1,2,4-oxadiazolyle, 1,2,3-oxadiazolyle, 1,3,4-oxadiazolyle, 1,2, 3-thiadiazolyle, 1,2, 4-thiadiazolyle, 1,3, 4-thiadiazolyle, 1,2, 4-triazolyle, 1,3, 4-triazolyle, 1,2,3-triazolyle et tétrazolyle; benzofuranyle, isobenzofuranyle, benzo[b]thiényle, indolyle, isoindolyle, 1H-indazolyle, benzimidazolyle, benzoxazolyle, benzisoxazolyle, benzothiazolyle, benzisothiazolyle, benzodioxolyle, 1H-benzotriazolyle, quinolyle, isoquinolyle, cinnolinyle, quinazolinyle, quinoxalinyle et phthalazinyle.

Les pseudopeptides de formule (I) peuvent être synthétisés en trois étapes à partir d'acides aminés naturels ou synthétiques et de réactifs commerciaux peu coûteux selon des techniques classiques connues de l'homme du métier ou décrites dans la littérature.

Les organogels peuvent également être obtenus selon des techniques classiques connues de l'homme du métier ou décrites dans la littérature. A titre d'exemple, ils peuvent être préparés par chauffage d'un pseudopeptide de formule (I) à reflux dans un solvant connu de l'homme du métier dans des proportions comprises entre 0,01 et 5%, avantageusement entre 0,2 et 2% en poids d'organogélateurs par rapport au solvant puis refroidissement.

Selon la structure et la densité de la matrice fibreuse et les propriétés recherchées pour les produits finaux, la concentration des pseudopeptides de formule (I) dans la solution peut varier de 1% à 70% en poids.

Dans un mode de réalisation avantageux du matériau isolant thermique selon l'invention, la susdite matrice fibreuse est une matrice d'origine végétale, animale, minérale, en polymère naturel ou synthétique, ou une matrice obtenue par leurs mélanges.

Dans un mode de réalisation plus avantageux, ladite matrice est notamment choisie parmi le groupe comprenant un panneau en fibre de bois, un matelas en fibre de bois, une matrice en coton, en laine, en laine minérale, en polystyrène, en polyuréthane, en polyisocyanate, ou en polyisocyanurate.

Dans un autre mode de réalisation avantageux du matériau isolant thermique selon l'invention, le pseudopeptide de formule (I) est choisi parmi ceux dans lesquels le groupe 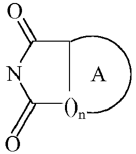 représente soit un groupe 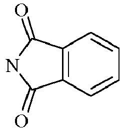 soit un groupe

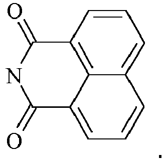

Dans un mode de réalisation plus avantageux du matériau isolant thermique selon l'invention, le pseudopeptide de formule (I) est choisi parmi ceux dans lesquels R représente soit -CH₂Ph, soit -C(CH₃)₃, soit - CH(CH3)2 et R₁ représente soit PhCH₂OCO-, soit CH₂=CH-CH₂OCO-.

Dans un mode de réalisation particulièrement avantageux, le matériau isolant thermique selon l'invention est obtenu par séchage d'un panneau en fibre de bois imprégné par une solution de pseudopeptides de formule (Ia)

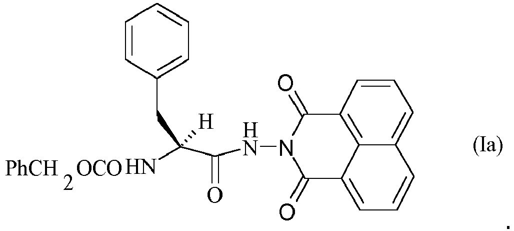

Dans un mode de réalisation particulièrement avantageux, le matériau isolant thermique selon l'invention est obtenu par séchage d'un matelas en fibre de bois imprégné par une solution de pseudopeptides de formule (Ia)

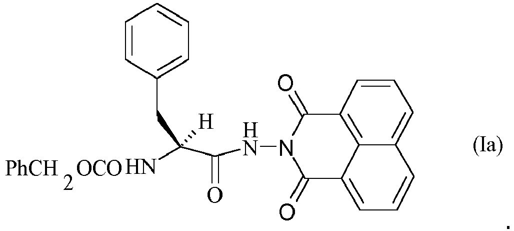

L'invention a aussi pour but de mettre à disposition un procédé de préparation d'un susdit matériau isolant thermique.

Ledit procédé comprend les étapes suivantes :
(i) la préparation d'une solution liquide de pseudopeptides de formule (I), en particulier de formule (Ia), dans un solvant ;
(ii) la mise en contact d'une matrice fibreuse susmentionnée avec la solution obtenue dans l'étape (i) jusqu'à que ladite matrice soit imprégnée totalement par ladite solution et atteigne sa capacité maximale d'absorption de ladite solution;
(iii) la formation d'un complexe organogel/matrice par refroidissement ;
(iv) l'extraction du solvant contenu dans ledit complexe organogel/matrice pour obtenir le susdit matériau isolant thermique.

L'extraction du solvant dans le complexe « organogel/matrice » peut être effectuée par toutes méthodes connues dans l'art, notamment par séchage en milieu CO₂ supercritique par des techniques classiques connues de l'homme du métier ou décrites dans la littérature, comme par exemple celle décrite dans la demande WO2010/133798.

L'invention concerne également un matériau isolant thermique susceptible d'être obtenu par un susdit procédé.

Un autre aspect de l'invention concerne l'utilisation d'une solution de pseudopeptides de formule (I), notamment de formule (Ia), pour améliorer l'hydrophobicité d'une matrice fibreuse, notamment l'hydrophobicité d'une matrice fibreuse ayant une faible conductivité thermique et une densité inférieure à 50kg/m³.

L'invention est illustrée plus en détail par l'exemple ci-après.

### Exemple 1 : Préparation d'un matériau de l'invention

Cet exemple illustre la préparation d'un matériau de l'invention obtenu par séchage d'un panneau en fibre de bois imprégné par une solution de pseudopeptides de formule (Ia).

### 1. Matériels et méthodes

### 1.1. Caractéristique du panneau en fibre de bois

Le panneau en fibre de bois utilisé dans l'exemple a une densité de 43 kg/m³ et se présente sous forme d'échantillons carrés de 5 x 5 cm de chaque côté.

### 1.2. Préparation de la solution de pseudopeptides de formule (Ia)

Une solution de pseudopeptides de formule (Ia) à 14,9% dans le 3-pentanol est préparée comme suit :
2,14g du composé de formule (Ia) ont été dissous à chaud dans 15 mL de 3-pentanol pour obtenir une solution de composé de formule (Ia) à 14,9% en masse. La solubilisation se fait pendant 2 minutes sous agitation dans un ballon placé dans un four à micro-ondes offrant la possibilité de travail en réacteur ouvert. La température de travail du four à micro-ondes est réglée à 100°C sous une puissance maximale de 150 Watt. Le ballon est équipé d'un système réfrigérant à eau pour condenser les vapeurs de solvant et maintenir la concentration de la solution.

La solution est maintenue à une température supérieure à la température de transition sol-gel, soit environ 100°C pour une solution à 14,9%.

### 1.3. Préparation du composite « panneau en fibre de bois/organogel »

Le panneau en fibre de bois est placé dans un moule en acier inoxydable de même dimension préalablement chauffé pour éviter tout choc thermique. La solution du composé de formule (Ia) dans le 3-pentanol maintenue à 100°C est ensuite coulée sur le panneau en fibre de bois. L'ensemble est refroidi à température ambiante jusqu'à gélification du gel. Le composite « panneau en fibre de bois/organogel » obtenu est ensuite extrait du moule.

### 1.4. Séchage du système composite « panneau en fibre de bois/organogel »

Le composite obtenu est séché selon le procédé de séchage par CO₂ supercritique décrit dans la demande WO2010/133798.

Ledit procédé est mis en œuvre dans un autoclave (8 cm de hauteur, 100 ml de volume) se composant d'un cylindre creux à double parois et de deux extrémités amovibles composées de frittés métalliques permettant le passage des fluides.

Le composite « panneau en fibre de bois/organogel » est placé dans un autoclave disposant d'un lit de billes recouvert de 0,5g de 3-pentanol et maintenu à 15°C. Après la fermeture de l'autoclave, le CO₂ préalablement refroidi à 4°C est introduit dans le réacteur sous une pression de 50 bars (5x10⁶Pa). La pression est ensuite portée à 90 bars (9x10⁶Pa) par injection de CO₂ à l'aide d'une pompe à membrane. La pression du premier séparateur est réglée à 50 bars (5x10⁶Pa) et celle du deuxième séparateur à 20 bars (2x10⁶Pa). Le débit de CO₂ est réglé à 400g/h. La température des éléments de séparation est maintenue à 20°C durant tout le procédé de séchage.

La température de l'autoclave est ensuite portée à 45°C afin de faire passer le système CO₂/3-pentanol en phase supercritique. Après 20 minutes, les vannes de sortie de l'autoclave et du dernier séparateur sont ouvertes.

L'extraction du 3-pentanol en continu pendant 4h avec un débit de CO₂ 400g/h permet l'obtention du composite « panneau en fibre de bois/aérogel » attendu.

### 2. Caractéristiques du produit « panneau en fibre de bois/aérogel »

Le produit final « panneau en fibre de bois/aérogel » obtenu a une densité de 183 Kg/m³.

La conductivité thermique du produit final est mesurée par la méthode du tricouche (Y. Jannot, G. Payet, A. Degiovanni, IJHMT 2009, 52, 1105-1111), complétée par une mesure au plan chaud centré (Y. Jannot, V. Felix, A. Degiovanni, Measurement Science and Technology 2010, 21, n° 035106).

La conductivité thermique dudit produit final mesurée à température ambiante d'environ 25°C est de 0,026 W/m/K.

Ledit produit final présente une meilleure résistance mécanique comparée à l'aérogel organique seul décrit dans la demande WO2010/133798 et au panneau en fibre de bois.

La densité du produit final, qui est d'environ 183 kg/m³, est nettement supérieure à celle de l'aérogel organique seul décrit dans la demande WO2010/133798 (2,83kg/m³) et celle du panneau en fibre de bois (43kg/m³).

Par ailleurs, ledit produit présente une meilleure hydrophobicité que le panneau en fibre de bois et proche de celle de l'aérogel décrit dans la demande WO2010/133798.

### Exemple 2 : Préparation d'un matériau de l'invention

Cet exemple illustre la préparation d'un matériau de l'invention obtenu par séchage d'un panneau en fibre de bois imprégné par une solution de pseudopeptides de formule (Ia).

### 1. Matériels et méthodes

### 1.1. Caractéristique du panneau en fibre de bois

Le panneau en fibre de bois utilisé dans l'exemple a une densité de 43 kg/m³ et se présente sous forme d'échantillons carrés de 5 x 5 cm de chaque côté.

### 1.2. Préparation de la solution de pseudopeptides de formule (Ia)

Une solution de pseudopeptides de formule (Ia) à 14,9% dans le 3-pentanol est préparée comme suit :
2,14g du composé de formule (Ia) ont été dissous à chaud dans 15 mL de 3-pentanol pour obtenir une solution de composé de formule (Ia) à 14,9% en masse. La solubilisation se fait pendant 2 minutes sous agitation dans un ballon placé dans un four à micro-ondes offrant la possibilité de travail en réacteur ouvert. La température de travail du four à micro-ondes est réglée à 100°C sous une puissance maximale de 150 Watt. Le ballon est équipé d'un système réfrigérant à eau pour condenser les vapeurs de solvant et maintenir la concentration de la solution.

La solution est maintenue à une température supérieure à la température de transition sol-gel, soit environ 100°C pour une solution à 14,9%.

### 1.3. Préparation du composite « panneau en fibre de bois/organogel »

Le panneau en fibre de bois est placé dans un moule en acier inoxydable de même dimension préalablement chauffé pour éviter tout choc thermique. La solution du composé de formule (Ia) dans le 3-pentanol maintenue à 100°C est ensuite coulée sur le panneau en fibre de bois. L'ensemble est refroidi à température ambiante jusqu'à gélification du gel. Le composite « panneau en fibre de bois/organogel » obtenu est ensuite extrait du moule.

### 1.4. Séchage du système composite « panneau en fibre de bois/organogel »

Le composite obtenu est séché selon le procédé de séchage par CO₂ supercritique décrit dans la demande WO2010/133798.

Ledit procédé est mis en œuvre dans un autoclave (8 cm de hauteur, 100 ml de volume) se composant d'un cylindre creux à double parois et de deux extrémités amovibles composées de frittés métalliques permettant le passage des fluides.

Le composite « panneau en fibre de bois/organogel » est placé dans un autoclave disposant d'un lit de billes recouvert de 3,5g de 3-pentanol et maintenu à 15°C. Après la fermeture de l'autoclave, le CO₂ préalablement refroidi à 4°C est introduit dans le réacteur sous une pression de 50 bars (5x10⁶Pa). La pression est ensuite portée à 90 bars (9x10⁶Pa) par injection de CO₂ à l'aide d'une pompe à membrane. La pression du premier séparateur est réglée à 50 bars (5x10⁶Pa) et celle du deuxième séparateur à 20 bars (2x10⁶Pa). Le débit de CO₂ est réglé à 400g/h. La température des éléments de séparation est maintenue à 20°C durant tout le procédé de séchage.

La température de l'autoclave est ensuite portée à 45°C afin de faire passer le système CO₂/3-pentanol en phase supercritique. Après 20 minutes, les vannes de sortie de l'autoclave et du dernier séparateur sont ouvertes.

L'extraction du 3-pentanol en continu pendant 4h avec un débit de CO₂ 400g/h permet l'obtention du composite « panneau en fibre de bois/aérogel » attendu.

### 2. Caractéristiques du produit « panneau en fibre de bois/aérogel »

Le produit final « panneau en fibre de bois/aérogel » obtenu a une densité de 183 Kg/m³.

La conductivité thermique du produit final est mesurée par la méthode du tricouche (Y. Jannot, G. Payet, A. Degiovanni, IJHMT 2009, 52, 1105-1111), complétée par une mesure au plan chaud centré (Y. Jannot, V. Felix, A. Degiovanni, Measurement Science and Technology 2010, 21, n° 035106).

La conductivité thermique dudit produit final mesurée à température ambiante d'environ 25°C est de 0,026 W/m/K.

Ledit produit final présente une meilleure résistance mécanique comparée à l'aérogel organique seul décrit dans la demande WO2010/133798 et au panneau en fibre de bois.

La densité du produit final, qui est d'environ 183 kg/m³, est nettement supérieure à celle de l'aérogel organique seul décrit dans la demande WO2010/133798 (2,83kg/m³) et celle du panneau en fibre de bois (43kg/m³) .
Par ailleurs, ledit produit présente une meilleure hydrophobicité que le panneau en fibre de bois et proche de celle de l'aérogel décrit dans la demande WO2010/133798.

### Exemple 3 : Préparation d'un matériau de l'invention

Cet exemple illustre la préparation d'un matériau de l'invention obtenu par séchage d'un matelas de fibres de bois brutes imprégnées par une solution de pseudopeptides de formule (Ia).

### 1. Matériels et méthodes

### 1.1. Caractéristique des fibres de bois brutes

Les fibres de bois utilisées dans l'exemple sont fines et brutes de défibrage industriel du bois.

### 1.2. Préparation de la solution de pseudopeptides de formule (Ia)

Une solution de pseudopeptides de formule (Ia) à 13% dans le 3-pentanol est préparée comme suit :
1,23 g du composé de formule (Ia) ont été dissous à chaud dans 10 mL de 3-pentanol pour obtenir une solution de composé de formule (Ia) à 13% en masse. La solubilisation se fait pendant 2 minutes sous agitation dans un ballon placé dans un four à micro-ondes offrant la possibilité de travail en réacteur ouvert. La température de travail du four à micro-ondes est réglée à 100°C sous une puissance maximale de 150 Watt. Le ballon est équipé d'un système réfrigérant à eau pour condenser les vapeurs de solvant et maintenir la concentration de la solution.

La solution est maintenue à une température supérieure à la température de transition sol-gel, soit environ 100°C pour une solution à 13%.

### 1.3. Préparation du composite « matelas de fibres de bois/organogel »

Des fibres de bois brutes sont placées et tassées dans un moule en aluminium de dimension 45 mm x 45 mm x 5 mm préalablement chauffé pour éviter tout choc thermique. La solution du composé de formule (Ia) dans le 3-pentanol maintenue à 100°C est ensuite injectée dans le moule, imprégnant ainsi le matelas de fibres de bois. L'ensemble est refroidi à température ambiante jusqu'à gélification du système. Le composite « matelas de fibres de bois/organogel » obtenu est ensuite extrait du moule.

### 1.4. Séchage du système composite « matelas de fibres de bois/organogel »

Le composite obtenu est séché selon le procédé de séchage par CO₂ supercritique décrit dans la demande WO2010/133798.

Ledit procédé est mis en œuvre dans un autoclave (8 cm de hauteur, 100 ml de volume) se composant d'un cylindre creux à double parois et de deux extrémités amovibles composées de frittés métalliques permettant le passage des fluides.

Le composite « matelas de fibres de bois/organogel » est placé dans un autoclave disposant d'un lit de billes recouvert de 3,5g de 3-pentanol et maintenu à 15°C. Après la fermeture de l'autoclave, le CO₂ préalablement refroidi à 4°C est introduit dans le réacteur sous une pression de 50 bars (5x10⁶Pa). La pression est ensuite portée à 90 bars (9x10⁶Pa) par injection de CO₂ à l'aide d'une pompe à membrane. La pression du premier séparateur est réglée à 50 bars (5x10⁶Pa) et celle du deuxième séparateur à 20 bars (2x10⁶Pa). Le débit de CO₂ est réglé à 400g/h. La température des éléments de séparation est maintenue à 20°C durant tout le procédé de séchage.

La température de l'autoclave est ensuite portée à 45°C afin de faire passer le système CO₂/3-pentanol en phase supercritique. Après 20 minutes, les vannes de sortie de l'autoclave et du dernier séparateur sont ouvertes.

L'extraction du 3-pentanol en continu pendant 4h avec un débit de CO₂ 400g/h permet l'obtention du composite « matelas de fibres de bois/aérogel » attendu.

### 2. Caractéristiques du produit « matelas de fibres de bois /aérogel »

Le produit final « matelas de fibres de bois /aérogel » obtenu a une densité de 143 Kg/m³.

La conductivité thermique du produit final est mesurée par la méthode du plan chaud centré (Y. Jannot, V. Felix, A. Degiovanni, Measurement Science and Technology 2010, 21, n° 035106).

La conductivité thermique dudit produit final mesurée à température ambiante d'environ 25°C est de 0,025 W/m/K.

## Revendications

1. Matériau isolant thermique obtenu par séchage d'une matrice fibreuse imprégnée par une solution de pseudopeptides de formule (I), 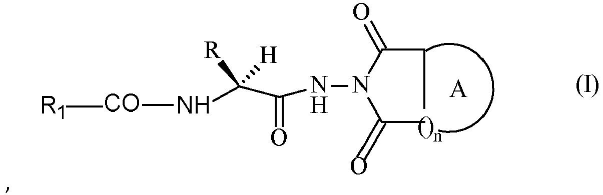 dans laquelle
- R représente une chaîne latérale d'un acide aminé naturel ou synthétique,
- R₁ représente soit un groupe (C₁-C₈)alkyle droit ou ramifié, soit un groupe (C₁-C₈)alcoxy droit ou ramifié, soit un groupe aryle, soit un groupe aryle(C₁-C₄)alkyle, soit un groupe aryloxy, soit un hétérocycle saturé ou insaturé, n = 1 ou 2 et
- A représente un groupe aromatique ou hétéroaromatique à un ou plusieurs cycles,
ladite matrice fibreuse ayant une faible conductivité thermique inférieure à 0,05 W/m/K et une densité inférieure à 50kg/m³,
la conductivité thermique étant mesurée par une méthode conformément à la description.

2. Matériau isolant thermique selon la revendication 1, **caractérisé en ce que** le pseudopeptide de formule (I) est choisi parmi ceux dans lesquels le groupe 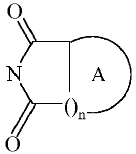 représente soit un groupe 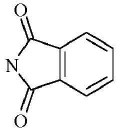 soit un groupe

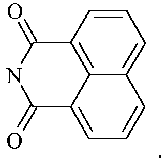

3. Matériau isolant thermique selon l'une quelconque des revendications 1 et 2, **caractérisé en ce que** le pseudopeptide de formule (I) est choisi parmi ceux dans lesquels R représente soit -CH₂Ph, soit -C(CH₃)₃, soit - CH(CH₃)₂ et R1 représente soit PhCH₂OCO-, soit CH₂=CH-CH₂OCO-.

4. Matériau isolant thermique selon l'une quelconque des revendications 1 à 3, **caractérisé en ce que** ladite matrice fibreuse est une matrice d'origine végétale, animale, minérale, en polymère naturel ou synthétique, ou une matrice obtenue par leurs mélanges.

5. Matériau isolant thermique selon la revendication 4, **caractérisé en ce que** ladite matrice est choisie parmi le groupe comprenant un panneau en fibre de bois, un matelas en fibre de bois, une matrice en coton, en laine, en laine minérale, en polystyrène, en polyuréthane, en polyisocyanate, ou en polyisocyanurate.

6. Matériau isolant thermique selon l'une quelconque des revendications 1 à 5, **caractérisé en ce que** ledit matériau est obtenu par séchage d'un panneau en fibre de bois imprégné par une solution de pseudopeptides de formule (Ia) ou d'un matelas en fibre de bois imprégné par une solution de pseudopeptides de formule (Ia)

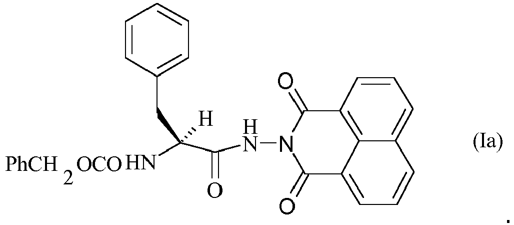

7. Procédé de préparation d'un matériau isolant thermique selon l'une quelconque des revendications 1 à 6, **caractérisé en ce que** ledit procédé comprend les étapes suivantes :
(i) la préparation d'une solution liquide de pseudopeptides de formule (I), en particulier de formule (Ia), dans un solvant ;
(ii) la mise en contact d'une matrice telle que décrite selon l'une quelconque des revendications 1, 4 et 5 avec la solution obtenue dans l'étape (i) jusqu'à ce que ladite matrice soit imprégnée totalement par ladite solution et atteigne sa capacité maximale d'absorption de ladite solution;
(iii) la formation d'un complexe organogel/matrice par refroidissement ;
(iv) l'extraction du solvant contenu dans ledit complexe organogel/matrice pour obtenir le susdit matériau isolant thermique.

8. Utilisation d'une solution de pseudopeptides de formule (I) tels que décrits selon l'une quelconque des revendications 1-3 et 6, pour améliorer l'hydrophobicité d'une matrice fibreuse.

## Patentansprüche

1. Wärmedämmstoff, gewonnen durch Trocknung einer faserigen Matrix, welche imprägniert ist mit einer Pseudopeptiden-Lösung der Formel (I), 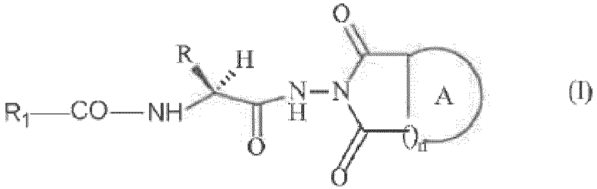 worin
- R für eine Seitenkette einer natürlichen oder synthetischen Aminosäure steht,
- R₁ entweder für eine lineare oder verzweigte Alkyl(C₁-C₈)-Gruppe, eine lineare oder verzweigte Alcoxy(C₁-C₈)-Gruppe, eine Arylgruppe, eine Alkyl(C₁-C₄)aryl-Gruppe, eine Aryloxygruppe, oder für eine gesättigte oder ungesättigte Heterozykle, n=1 oder 2, steht und
- A für einen mono- oder polyzyklischen Aromaten oder Heteroaromaten steht, wobei die faserige Matrix eine niedrige Wärmeleitung unter 0,05 W/m/K und eine Dichte unter 50 kg/m³ aufweist,
wobei die Wärmeleitung über ein Verfahren gemäß der Beschreibung gemessen wird.

2. Wärmedämmstoff nach Anspruch 1, **dadurch gekennzeichnet, dass** das Pseudopeptid der Formel (I) aus denen ausgewählt wird, in welchen die Gruppe 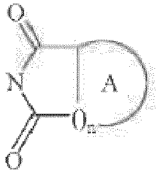 entweder für eine Gruppe 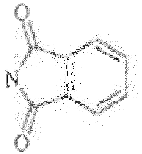 oder eine Gruppe 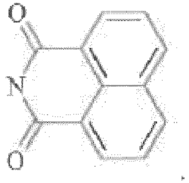 steht.

3. Wärmedämmstoff nach einem der Ansprüche 1 und 2, **dadurch gekennzeichnet, dass** das Pseudopeptid der Formel (I) aus denen ausgewählt wird, in welchen R entweder für -CH₂Ph, -C(CH₃)₃ oder -CH(CH₃)₂ steht und R₁ entweder für PhCH₂OCO- oder CH₂=CH-CH₂OCO- steht.

4. Wärmedämmstoff nach einem der Ansprüche 1 bis 3, **dadurch gekennzeichnet, dass** die faserige Matrix eine Matrix pflanzlichen, tierischen, mineralischen Ursprungs, oder aus natürlichem oder synthetischem Polymer besteht oder eine durch Mischen dieser Stoffe gewonnene Matrix ist.

5. Wärmedämmstoff nach Anspruch 4, **dadurch gekennzeichnet, dass** die Matrix ausgewählt ist aus der Gruppe bestehend aus einer Holzfaserplatte, einer Holzfasermatte, einer Matrix aus Baumwolle, Wolle, Mineralwolle, Polystyrol, Polyurethan, Polyisocyanat oder Polyisocyanurat.

6. Wärmedämmstoff nach einem der Ansprüche 1 bis 5, **dadurch gekennzeichnet, dass** der Stoff durch Trocknung einer mit einer Pseudopeptiden-Lösung der Formel (la) imprägnierten Holzfaserplatte oder einer mit einer Pseudopeptiden-Lösung der Formel (la) imprägnierten Holzfasermatte gewonnen wird

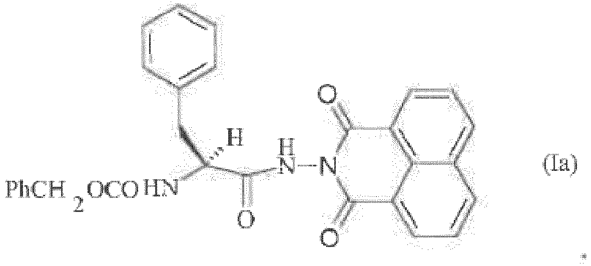

7. Verfahren zur Herstellung eines Wärmedämmstoffes nach einem der Ansprüche 1 bis 6, **dadurch gekennzeichnet, dass** das Verfahren die folgenden Schritte umfasst:
(i) die Zubereitung einer flüssigen Pseudopeptiden-Lösung der Formel (I), insbesondere der Formel (la), in einem Lösungsmittel;
(ii) die Kontaktierung einer Matrix wie beschrieben nach einem der Ansprüche 1, 4 und 5 mit der in Schritt (i) gewonnenen Lösung, bis die Matrix vollständig mit der Lösung imprägniert ist und ihre maximale Absorptionsfähigkeit der Lösung erreicht;
(iii) die Bildung eines Organogel-/Matrixkomplexes durch Kühlung;
(iv) das Extrahieren des im Organogel-/Matrixkomplex enthaltenen Lösungsmittels zur Gewinnung des Wärmedämmstoffes.

8. Verwendung einer Pseudopeptiden-Lösung der Formel (I) wie beschrieben nach einem der Ansprüche 1-3 und 6 zur Steigerung der Hydrophobizität einer faserigen Matrix.

## Claims

1. Heat insulation material obtained by drying a fibrous matrix impregnated with a solution of pseudopeptides of formula (I), 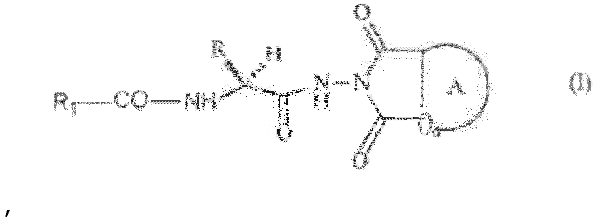 in which
- R represents a side chain of a natural or synthetic amino acid,
- R₁ represents either a linear or branched (C₁-C₈)alkyl group, or a linear or branched (C₁-C₈)alkoxy group, or an aryl group, or an aryl(C₁-C₄)alkyl group, or an aryloxy group, or a saturated or unsaturated heterocycle, n = 1 or 2 and
- A represents an aromatic or heteroaromatic group with one or more rings, said fibrous matrix having a low thermal conductivity less than 0.05 W/mK and a density less than 50kg/m³,
the thermal conductivity being measured by a method according to the description.

2. Heat insulation material according to claim 1, **characterized in that** the pseudopeptide of formula (I) is selected from those in which the 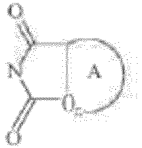 group represents either a 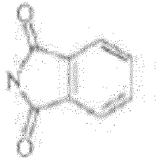 group or a 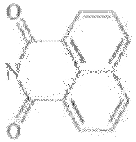 group.

3. Heat insulation material according to any one of claims 1 and 2, **characterized in that** the pseudopeptide of formula (I) is selected from those in which R represents either -CH₂Ph, or -C(CH₃)₃, or -CH(CH3)2 and R1 represents either PhCH₂OCO-, or CH₂=CH-CH₂OCO-.

4. Heat insulation material according to any one of claims 1 to 3, **characterized in that** said fibrous matrix is a matrix of plant, animal, mineral origin, made from natural or synthetic polymer, or a matrix obtained by mixtures thereof.

5. Heat insulation material according to claim 4, **characterized in that** said matrix is selected from the group comprising a fibreboard, a wood fibre blanket, a matrix made from cotton, wool, mineral wool, polystyrene, polyurethane, polyisocyanate, or polyisocyanurate.

6. Heat insulation material according to any one of claims 1 to 5, **characterized in that** said material is obtained by drying a fibreboard impregnated with a solution of pseudopeptides of formula (Ia) or a wood fibre blanket impregnated with a solution of pseudopeptides of formula (Ia)

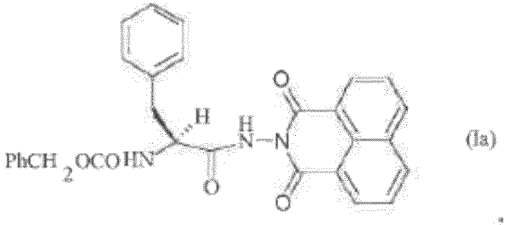

7. Process for preparing a heat insulation material according to any one of claims 1 to 6, **characterized in that** said process comprises the following steps:
(i) preparing a liquid solution of pseudopeptides of formula (I), in particular of formula (Ia), in a solvent;
(ii) bringing a matrix as described according to any one of claims 1, 4 and 5 into contact with the solution obtained in step (i) until said matrix is fully impregnated with said solution and reaches its maximum absorption capacity of said solution;
(iii) forming an organogel/matrix complex by cooling;
(iv) extracting the solvent contained within said organogel/matrix complex in order to obtain the abovementioned heat insulation material.

8. Use of a solution of pseudopeptides of formula (I) as described according to any one of claims 1-3 and 6 in order to improve the hydrophobicity of a fibrous matrix.
